(19) Europäisches Patentamt
European Patent Office
Office européen des brevets

(11) **EP 1 331 855 B1**

(12) **EUROPEAN PATENT SPECIFICATION**

(45) Date of publication and mention
of the grant of the patent:
**17.04.2013 Bulletin 2013/16**

(51) Int Cl.:
*A23K 1/16* (2006.01)          *A23K 1/18* (2006.01)
*A61K 31/34* (2006.01)          *A61K 31/355* (2006.01)
*A61K 31/385* (2006.01)

(21) Application number: **01988317.2**

(22) Date of filing: **30.10.2001**

(86) International application number:
**PCT/US2001/048495**

(87) International publication number:
**WO 2002/045525 (13.06.2002 Gazette 2002/24)**

(54) **AGED COMPANION PET DIET**

DIÄT FÜR ÄLTERE HAUSTIERE

COMPOSITION POUR ANIMAUX DE COMPAGNIE AGÉS

(84) Designated Contracting States:
**AT BE CH CY DE DK ES FI FR GB GR IE IT LI LU MC NL PT SE TR**
Designated Extension States:
**RO**

(30) Priority: **31.10.2000 US 244510 P**
**28.11.2000 US 253447 P**
**06.08.2001 US 922632**
**16.10.2001 US 978127**

(43) Date of publication of application:
**06.08.2003 Bulletin 2003/32**

(73) Proprietor: **Colgate-Palmolive Company**
**New York NY 10022-7499 (US)**

(72) Inventors:
• **ZICKER, Steven, Curtis**
**Lawrence, KS 66049 (US)**
• **WEDEKIND, Karen, J.**
**Meriden, KS 66512 (US)**

(74) Representative: **Jenkins, Peter David et al**
**Page White & Farrer**
**Bedford House**
**John Street**
**London WC1N 2BF (GB)**

(56) References cited:
**WO-A-00/44375          WO-A-01/58271**
**CA-A- 2 285 490          US-A- 5 883 083**
**ZA-A- 9 605 149**

• **BRANAM J E: "DIETARY MANAGEMENT OF GERIATRIC DOGS AND CATS" VETERINARY TECHNICIAN, VETERINARY LEARNING SYSTEMS, PRINCETON JUSTICE, NJ,, US, vol. 8, no. 10, 1987, pages 501-503, XP000978775 ISSN: 8750-8990**

• **KOLB E ET AL: "ZUM BEDARF AN VITAMINEN UND AN ASCORBINSAEURE BEIM HUND MIT BEMERKUNGEN ZUR PUBLIKATION VON M. TOREL TU51 785-490 1996" TIERAERZTLICHE UMSCHAU, KONSTANZ, DE, vol. 52, no. 12, 1997, pages 728-733, XP000978781 ISSN: 0049-3864**

EP 1 331 855 B1

**Description**

Background of the Invention

Companion animals such as dogs and cats seem to suffer from aging problems. Some of these are manifested in commonplace sayings. One of these is "You can't teach an old dog new tricks". This saying arises from the observation that as dogs age, their mental capacity seems to diminish as well as physical abilities. Mental activities associated with thinking learning and memory seem to be lessened (Cummings BJ, Head E, Ruehl W, Milgram NW Cotman CW 1996: The canine as an animal model of human aging and dementia; Neurobiology of aging 17:259-268). Additionally, behavioral change can be manifested in the aging animals in association with the changes in mental capacity. Many causes have been assigned to this lessening of capacity. WO 00/44375 discloses antioxidant compositions for companion animals to prevent a treat a disorder of oxidative stress.

It has now been demonstrated that the presence of significant levels of at least one antioxidant in the diet of an aged companion pet inhibits the deterioration of the mental capacity of an aging companion pet.

Summary of the Invention

In accordance with the invention, there is a companion pet diet meeting ordinary nutritional requirements of an aged pet and further comprising a sufficient amount of an antioxidant or mixtures thereof for use in inhibiting the deterioration of the mental capacity of an aged companion pet.

In further accordance with the invention is a companion aged pet diet meeting ordinary nutritional requirements of the aged pet and further comprising an antioxidant selected from the group consisting of Vitamin E, vitamin C, alpha-lipoic acid, 1-carnitine and any mixture thereof in quantities sufficient to inhibit the deterioration of the mental capacity of an aged companion pet.

A still further aspect of the invention is a companion pet diet for use in increasing the mental capacity of an aged companion pet, which use in comprises an amount of an antioxidant or mixture thereof sufficient to increase the mental capacity.

Detailed Description of the Invention

The diet fed to the aging companion pet, for example canine and feline is the standard normal diet fed to an animal of that age. Below is a typical diet for a canine of at least 7 years of age.

**Table 1**

| Component | Target |
|---|---|
| Protein (% of dry matter) | 19.5 |
| Fat (% of dry matter) | 10 |
| Phosphorous (% of dry matter) | 0.5 |
| Sodium (% of dry matter) | 0.2 |

Adding significant quantities of an antioxidant and mixtures thereof to the companion pet diet can bring about significant and demonstrative changes in the behavior, particularly the mental capacity, as specifically shown by problem-solving capacity, in an aged pet. The term, aged, is intended to mean, in general, a canine of at least seven years and a feline of at least seven years.

The loss of mental capacity for canines and felines has been observed for a number of years. This loss of mental capacity is manifested in numerous ways. For a canine, for example, it can be manifested as disorientation, house soiling, altered sleep-wake patterns, decreased interaction with family members and pets, and inability to learn or concentrate. These conditions can be manifested in felines as well. Alzheimer's, as exhibited in man, is not found in canines and felines.

Many theories have been advanced for this loss in mental capacity. To date, the inventors are unaware of any dietary course of action, which inhibits this loss of mental capacity or can actually bring about a positive change in mental capacity as measured by an objective parameter.

The inventors have succeeded in accomplishing this. By using the diet of their invention, it has been demonstrated that aging dog's deteriorating mental capacity can be inhibited and, as measured by problem-solving capability can be enhanced. Essentially the deterioration of mental capacity can be reversed. The mental capacity of an aged pet in need of such treatment can have its mental capacity increased. Problem-solving, as demonstrated by memory and learning ability can be improved. Overall mental alertness can be enhanced. Age related cognitive decline can be slowed. With respect to Cognitive Dysfunction Syndrome, its progress can be slowed in aged dogs and clinical signs associated with this syndrome can be controlled. Prophylaxis where appropriate and pets in need of these component(s) are the target group.

The component in the diet, which accomplishes this, is an antioxidant or mixture thereof. An antioxidant is a material that quenches a free radical. Examples of such materials include foods such as Ginkgo Biloba, citrus pulp, grape pomace, tomato pomace, carrot and spinach, all preferably dried as well as various other materials such as beta-carotene, selenium, coenzyme Q10 (ubiquinone), lutein, tocotrienols, soy isoflavones, S-adenosylmethionine, glutathione, taurine, N-acetylcysteine, Vitamin E, Vitamin C, alpha-lipoic acid, 1-carnitine and the like. Vitamin E can be administered as a tocopherol or a mixture of tocopherols and various derivatives thereof such as esters like vitamin E acetate, succinate, palmitate, and the like. The alpha form is preferable but beta, gamma and delta forms can be included. The d form is preferable but racemic mixtures are acceptable. The forms and derivatives will function in a Vitamin E like activity after ingestion by the pet. Vitamin C can be administered in this diet as ascorbic acid and its various derivatives thereof such as calcium phosphate salts, cholesteryl salt, 2- monophosphate, and the like which will function in a vitamin C like activity after ingesting by the pet. They can be in any form such as liquid, semisolid, solid and heat stable form. Alpha-lipoic acid can be administered into the diet as alpha lipoic acid or as a lipoate derivative as in US Patent 5,621,117, racemic mixtures, salts, esters or amides thereof. L-carnitine can be administered in the diet and various derivatives of carnitine such as the salts such as the hydrochloride, fumarate and succinates, as well as acetylated carnitine, and the like can be used.

The quantities administered in the diet, all as wt % (dry matter basis) of the diet, are calculated as the active material, per se, that is measured as free material. The maximum amounts employed should not bring about toxicity. At least about 100 ppm or at least about 150 ppm of Vitamin E can be used. A preferred range of about 500 ppm to about 1,000 ppm can be employed. Although not necessary a maximum of about 2000 ppm or about 1500 ppm is generally not exceeded. With respect to Vitamin C at least about 50 ppm is used, desirably at least about 75 ppm and more desirably at least about 100 ppm. A nontoxic maximum can be employed. The quantity of alpha-lipoic acid can vary from at least about 25 ppm, desirably at least about 50 ppm, more desirably about 100 ppm. Maximum quantities can vary from about 100 ppm to 600 ppm or to an amount which remains non toxic to the pet. A preferred range is from about 100 ppm to about 200 ppm. For 1-carnitine about 50 ppm, desirably about 200 ppm, more desirably about 300 ppm for canines are a useful minimum. For felines, slightly higher minimums of 1-carnitine can be employed such as about 100 ppm, 200 ppm, and 500 ppm. A nontoxic maximum quantity can be employed, for example, less than about 5,000 ppm. For canines, lower quantities can be employed, for example, less than about 5,000 ppm. For canines a preferred range is about 200 ppm to about 400 ppm. For felines a preferred range is about 400 ppm to about 600 ppm.

Beta-carotene at about 1-15 ppm can be employed.

Selenium at about 0.1 up to about 5 ppm can be employed.

Lutein at least about 5 ppm can be employed.

Tocotrienols at least about 25 ppm can be employed.

Coenzyme Q10 at least about 25 ppm can be employed.

S-adenosylmethionine at least about 50 ppm.

Taurine at least about 1000 ppm can be employed.

Soy isoflavones at least about 25 ppm can be used.

N-acetylcysteine at least about 50 ppm can be used.

Glutathione at least about 50 ppm can be used.

Ginkgo Biloba at least 50 ppm of extract or 1% of diet can be used.

The following are raw ingredients that are high in ORAC (Oxygen radical absorbing capacity) content. When added to the diet at 1% inclusions (for a total of 5% substitution for a low ORAC ingredient such as corn) they increased the ORAC content of the overall diet and increased the ORAC content of the plasma of the animals which ate the diet containing these components. Preferably, any ingredient with an ORAC content >25 umole of Trolox equivalents per gram of dry matter could be used if added at 1% combination with four other 1% ingredients for a total of 5% addition to the diet.

Spinach pomace

Tomato pomace

Citrus Pulp

Grape pomace

Carrot granules

Broccoli

Green tea

Ginkgo Biloba

Corn gluten meal

## Example 1

All dogs were beagles and 7 years old or greater. The nutritional components of the control and test diet were approximately the same as the typical diet disclosed earlier in Table 1. However, the control diet contained 59 ppm Vitamin E and < 32 ppm Vitamin C. The test diet had 900 ppm Vitamin E and 121 ppm Vitamin C, 260 ppm 1-carnitine and 135 ppm alpha lipoic acid.

Twelve--aged beagle dogs were given a battery of baseline problem solving tasks prior to placement into either a control or enriched test diet group. The aged animals were equally matched with respect to learning (discrimination reversal) and memory (delayed non-match to position [DNMP] and delayed non-match to sample [DNMS]). A T-test was used to compare the two groups of dogs on baseline learning of the discrimination reversal learning, DNMP, and DNMS tasks. The results were non-significant. Thus, dogs were equally matched on the basis of cognition prior to diet intervention. Approximately 1 month after starting the diet, the first problem-solving task given to dogs was a landmark discrimination learning task, which is a test of spatial attention (Milgram et al., 1999 Milgram, N.W., Adams, B., Callahan, H., Head, E., Mackay, B., Thirlwell, C., & Cotman (1999), C.W. Landmark Discrimination Learning in the Dog. Learning & Memory, 6:54-61).

Landmark discrimination learning requires subjects to select a particular object based on proximity to an object. The initial learning, however, is based on the dogs' ability to learn an object discrimination task. We have previously found that the effects of age on discrimination learning depends on task difficulty, and we have evidence to indicate that landmark discrimination learning is markedly impaired in aged dogs.

When aged animals on the enriched test diet and control diet were compared on the landmark discrimination learning tasks, there was a highly significant difference between the groups. ($p<02$). Animals on the enriched diet acquired the task with fewer errors than did the animals on the control diet. Whereas all 6 of the animals on the enhanced diet were able to meet the learning criterion within 40 sessions, only 3 of the 6 animals on the control diet were able to meet the learning criterion. In addition, the 3 dogs that were able to solve the problem committed more errors than dogs receiving the enriched diet.

Dogs in the control and enriched test diet group, after completing landmark discrimination learning, have been tested on an oddity task. This task involves presenting dogs with 3 objects covering all 3 food wells. Two of these objects are identical and one is different. To obtain a food reward, dogs must select the odd object. Dogs on the enriched test diet learned this task with significantly fewer errors than dogs fed the control diet ($p<.003$ for all 4 oddity test scores combined).

## Example 2

Beagles (n=28) were pre-trained on a size discrimination task and ranked according to the errors to criteria in learning this task. The dogs were then stratified by rank into groups of three and randomly assigned to one of three diets based on prior cognition scores. All dogs enrolled in this study were greater than 7 years of age. Dogs were placed on one of three dry foods varying in vitamin E content and initiated on a landmark discrimination protocol. The Vitamin E content and other components are listed in Table 2 below.

**Table 2**

| Diet No. | Vitamin E | Vitamin C | L-Carnitine | Lipoic Acid |
|---|---|---|---|---|
| 1 | 799 ppm | 114 ppm | 294 ppm | 135 ppm |
| 2 | 172 ppm | < 32 ppm | 42 ppm | None added |
| 3 | 57 ppm | < 32 ppm | 13 ppm | None added |

The landmark discrimination protocol consisted of three phases of testing (landmark 0, 1, 2) which required dogs to reach a passing criteria (8/10 correct for two days in a row followed by 7/10 average for next three days) before moving to the next phase of the test. Each dog was allowed 40 days with 10 trials per day to learn each phase. Repeated MANOVA revealed a significant overall effect of diet on errors to criteria scores ($P<0.05$). Regression analysis of the summation of errors for landmark 1+2 versus the Vitamin E content of the diet revealed a significant ($P<0.05$) regression slope with dogs on the highest E diet making the least errors (mean = 65) and those on the lowest E diet making the most errors (mean = 170).

### Example 3

30 adult, random source, dogs were utilized for this study. Dogs were at least 10 months of age, not pregnant, not lactating and of reasonable body weight prior to start of test. Animals were randomized into 5 groups for dietary treatment with 3 males and 3 females per each group.

All dogs were fed a control food (0 ppm dl-alpha-lipoic acid added) that met or exceeded all recommendations for nutrients as proposed by the American Association of Feed Control Officials (AAFCO 2000) during a 2 week prefeeding period (Table 1). Following the prefeeding period dogs were randomized into 5 treatment groups with one of the following dl-alpha lipoic acid target-inclusions (dry matter basis): 0 ppm, 150 ppm, 1500 ppm, 3000 ppm, 4500 ppm. In all diets, control and alpha lipoic acid, Vitamin E was added and was present at a level of 600-1000 International Units and Vitamin C was added at levels of 100-200 ppm.

Test foods were the sole source of nutrients except for water. Fresh water was provided ad libitum. After dogs were selected and initial body weights taken, a food dose was calculated for each dog based on the expected ME of the food. Initial food dose calculations were based on the maintenance energy requirement (MER) for the dog modified by a factor to account for normal activity as calculated by the following formula:

$$\text{MER (kcal/day)} = 1.6 \text{ X RER (Resting Energy Requirement)}$$

Where: RER (kcal/day) = 70 X body weight (kg)0.75

Dogs were weighed weekly and had food doses adjusted as needed in order to feed enough food to maintain their optimal body weight. Optimal body weight was determined to be 3 on a 5 point scale. If a dog did not maintain body weight within -10% of initial body weight, after adjustment of food dose, it was removed from the study. All measures of body weight and food intake were recorded.

Samples were ground and $0.100 \pm 0.001$g of sample was extracted twice into 5.0 mL phosphate buffer (10mM $Na_2HPO_4$, 2mM ethylenediaminetetraacetatic acid (EDTA), 0.9% NaCl, pH 7.4)[4]. 250 $\mu$L of extract was placed into a 5 mL glass centrifuge tube with a Teflon lined cap. 15 $\mu$L EDTA solution (100mM EDTA, adjusted to pH 7.8 with ~1M NaOH) and 50 $\mu$L freshly prepared 5mM dithioerythritol (DTE) were added. The solutions were vortexed and incubated at room temperature for 5 minutes. Then 10 $\mu$L of 1 M $H_3PO_4$ and 2.0 mL ,diethyl ether were added. The tubes were capped, vortexed, and centrifuged at 1500 x g for 3 minutes at room temperature. The ether layer was transferred to a separate 5 mL glass centrifuge tube, while the aqueous layer was extracted twice more with 1.5 mL ether. All extractions from the same sample were combined. The extracts are then dried in a nitrogen evaporator in a water bath at room temperature. At this point, the samples were capped and frozen overnight.

The dried extracts were then thawed and reconstituted with 70 $\mu$L SDS/EDTA solution (0.11% sodium dodecyl sulfate (SDS), 15 mM EDTA, 0.9% NaCl) and 5 $\mu$L freshly prepared 1mM DTE. 50 $\mu$L of freshly prepared $NaBH_4$ was then added to each tube. The tubes were vortexed and incubated at room temperature for 10 minutes. After 10 minutes, the samples were frozen at -70° C. Before the solutions were thawed, 20 $\mu$L 2M HCl was added. After the solutions were thawed, 800 $\mu$L 100mM $NH_4HCO_3$ was added. The solutions are vortexed and 5 $\mu$L of 100mM momobromobimane in acetonitrile solution (mBBr) was added. The solutions were then incubated in the dark for 90 minutes at room temperature.

Excess mBBr and the DTE derivative were removed from the samples after incubation by extraction with 1.5 mL dichloromethane. The aqueous layer was placed on the HPLC. The lipoic acid was separated using a mobile phase that consisted of 30% acetonitrile, 1% acetic acid, adjusted to pH 3.95 with ~2M $NH_4OH$ and was pumped at a flow rate of 1.0 mL/min with an isocratic elution for 15 minutes per injection. This preparation assumes that the density of the extruded food is equal to 1g/mL.

Blood was collected aseptically for complete blood count, and blood biochemistry analysis 2 weeks prior to start and again at 0, 28, 56, 84, 112, 140 and 168 days of the study. In addition, 15 ml of whole blood was collected for isolation of lymphocytes at day 0, 28 and 84 of the dietary intervention.

Heparainzed whole blood was layered onto a 50 ml Accuspin conical centrifuge tube (Sigma Chemical) and an equal volume of Phosphate buffered saline (PBS) was added. Samples were centrifuged at at 700g for 30 minutes without brake. The monocyte layer was harvested, transferred to a 15 ml conical centrifuge tube, resuspended in 1-3 ml of PB, and centrifuged as before (First wash). A second wash was performed as the first wash. Finally, cells were harvested and suspended in perchloric acid (10%w/v) and frozen at -70C until analysis.

Samples were transferred from -70°C freezer into a cooler with dry ice in it. Vials were centrifuged at 12,000 rpm for 5 minutes in a refrigerated centrifuge. An aliquot of supernatant for glutathione (GSH) analysis was transferred to a conical test tube.

Derivatization of the acid soluble extracts was by the method of Reed and coworkers (Fariss et al) as modified by Jones (Jones et al)

Briefly, 150 μl extract or external standards were added into a 1.5 ml eppendorf tube followed by addition of 20 μl γ-glu-glu internal standard and 50 μl IAA added followed by mixing. The solution was adjusted to pH -10 (purple color) by using KOH-KHCO$_3$ working solution. Solutions were incubated 1 hr. under room temperature in the dark. Sanger's reagent was added at the same volume as of the total volume and the solution was incubated overnight (20hrs) in the dark at room temperature.

After incubation, the solution was centrifuged at 12000 rpm for 5 minutes with the supernatant transferred into another 1.5 ml eppendorf tube. 200 μl supernatant was added into an amber autovial which had a 300 μl inlet, fix the top with a crimper for HPLC analysis.

Solvents and separation conditions were as described (Fariss, Jones). Levels of GSH and GSSG were quantified relative to authentic standards. Gamma-glutamyl-glutamate was used as an internal standard to assess derivatization efficiency.

Comparison of values for clinical chemistry, hematology and body weights vs baseline were analyzed by way of paired t-test on SAS for windows with significance set at $P<0.05$. Means of values at each measured time point were separated by a one-way ANOVA with significance set at $P<0.05$. The difference in GSH:GSSG between day 84 and baseline were analyzed between groups by way of SAS for windows in a one-way ANOVA with significance set at $P<0.05$.

**Results**

Concentrations of lipoic acid (ppm) in food as determined over 7 successive assays (0, 28, 56, 84, 112, 140, 168 days) were within the range of expected assay sensitivity and production parameters typically encountered at our facility (Table 1).

The food intake data were unremarkable. Most animals in all groups ingested more food at 6 months, on average, than at the beginning of the study. Body weight data were unremarkable except that some weight loss occurred initially in the 4500 ppm inclusion group but that change appeared to reversed by 6 months time. Body condition scores did not appear to be affected by this minor loss of weight.

The routine physical examinations did not reveal any evidence of nutrition related abnormalities or d1-alpha-lipoic acid toxicity. All animals in the study population remained normal during the entire course of the study. Occasional vomiting was observed in several animals during the course of the study; however, a trend was not observed that would lead one to the conclusion that the vomiting may be attributable to lipoic acid. One animal, in the highest inclusion group, was dropped from the study at day 21 for weight loss and leukocytosis. The leukocytosis in this animal had not resolved by the end of the study and is suspected to be attributable to some other disease process.

When serum biochemistry values for days 28, 56, 84, 112, 140, and 168 were compared with the initial values for the same group of dogs, several statistical differences were noted, however, none of these were considered biologically significant because these values were within or very near the laboratory reference range and consistent trends over months were noted. Comparisons between the controls and the other treatment groups at each time period also revealed several statistical differences, however, none of these were considered biologically significant because these values were within or very near the clinical laboratory reference ranges and no trends were present.

When the hematology values for days 28, 56, 84, 112, 140 and 168 were compared with the initial values for the same group of dogs, several statistical differences were noted; however, none of these were considered biologically significant because these values were within or very near the laboratory reference range and not trends were present. Comparison between the controls and the other treatment groups at each time period revealed several statistical differences; however, none of these were considered biologically significant because these values were within or very near the clinical laboratory reference ranges and no trends were present.

*GSH: GSSG ratio*

The change in GSH:GSSG ratio over 84 days of feeding displayed a significant overall effect of diet (P=.024) with all supplemented groups having an increase in the ratio (Table 2). ANOVA revealed a significant difference, compared to the basal food, for the lowest and highest inclusions, however, the largest numerical increase was in the lowest inclusion level. That is to say, the changes in the GSH:GSSG ratio for the highest and lowest inclusion were significantly different from the change observed over this same time period in the basal food. Ratios for 4 points could not be determined at day 84 as no GSSG was detectable in any of these samples (1 control, 3 treatment groups). As such, the values for supplemented groups may have displayed even higher ratios of GSH:GSSG if the assay had been sensitive enough to detect the low levels of GSSG at day 84.

**Table 1**

| Inclusion Rate (ppm) | Average | Standard Deviation | Percent Target |
|---|---|---|---|
| 0 | 24 | 17 | NA |
| 150 | 151 | 13 | 101 |
| 1500 | 1471 | 113 | 98 |
| 3000 | 2869 | 250 | 96 |
| 4500 | 4176 | 642 | 93 |

**Table 2**

| Change in mean ratio of GSH:GSSG from day 0 to day 84 in dogs consuming d1-alpha lipoic acid in an extruded food. | | | |
|---|---|---|---|
| Inclusion | Difference in GSH:GSSG ratio-d 0 to d 84 compared to baseline food | N | P value |
| 0 ppm | -9.2 $\pm$ 26 | 5* | NA |
| 150 ppm | 70 $\pm$ 20 | 6 | .003 |
| 1500 ppm | 24 $\pm$ 7 | 6 | .16 |
| 3000 ppm | 10 $\pm$ 4 | 4* | .46 |
| 4500 ppm | 50 $\pm$ 36 | 4* | .03 |
| *1 dog in the control and 4500 ppm group had no detectable GSSG at day 84 while 2 dogs in the 3000 ppm group had no detectable GSSG at day 84. | | | |

Further observations with respect to alpha lipoic acid are applicable. Chronic feeding of alpha lipoic acid in diet is safe and effective. It improves the reduced glutathione (GSH) to oxidized glutathione (GSSG) ratio. The chronic administration of alpha lipoic acid in the diet can be for periods of one, two, three, four, five, or six months minimum up through a period of one, two, three, four, five years or even more including the lifetime of the animal. The alpha lipoic acid functions without any special protection in the diet such as encapsulation and need not be present in the diet in a unit dosage form such as those used in pharmaceuticals for example, tablet, pill, capsule and the like. The lipoic acid is provided in the diet in a minimum of about 25, 50, 75, or 100 ppm of diet. The uppermo st range is just below its toxic level, all the way down to about 400, 300, or 200 ppm of diet. Generally, one does not go beyond about 6 or 7 mg/kg body weight of animal per day, more generally not above about 5. The alpha lipoic acid improves antioxidant defense capabilities as well as improves the animal's ability to resist oxidative damage. All this is done with the proper quantities of other antioxidants present such as Vitamin E and Vitamin C. This demonstrates that the action of alpha lipoic acid is beyond that of Vitamin C and/or Vitamin E.

**Claims**

1.  A companion pet diet meeting ordinary nutritional requirements for an aged pet, wherein the diet further comprises a sufficient amount of an antioxidant or mixture thereof, for use in inhibiting the deterioration of the mental capacity of an aged companion pet.

2.  A companion pet diet meeting ordinary nutritional requirements for an aged pet, wherein the diet further comprises a sufficient amount of an antioxidant or mixture thereof, for use in increasing the mental capacity of an aged companion pet,

3.  A diet for use in accordance with claim 1 or claim 2 wherein the pet is a canine.

4.  A diet for use in accordance with claim 3 wherein the canine is at least seven years.

**5.** A diet for use in accordance with claim 1 or claim 2 wherein the pet is a feline.

**6.** A diet for use in accordance with claim 5 wherein the feline is at least seven years.

**7.** A diet according to any preceding claim wherein the antioxidant is selected from the group consisting of Vitamin C, alpha-lipoic acid, 1-carnitine or mixtures thereof.

**8.** A diet for use in accordance with any preceding claim, wherein Vitamin E is present in the diet in at least about 100 ppm of the diet.

**9.** A diet for use in accordance with claim 7 or claim 8, wherein at least about 50 ppm of Vitamin C are in the diet.

**10.** A diet for use in accordance with any of claims 7 to 9, wherein at least about 25 ppm of alpha-lipoic acid are in the diet.

**11.** A diet for use in accordance with any of claims 7 to 10, wherein at least about 50 ppm of 1-carnitine are present in the diet.

**12.** A diet for use in accordance with any of claims 7 to 11, wherein the diet comprises at least about 100 ppm of Vitamin E, at least about 50 ppm of Vitamin C, at least about 25 ppm of alpha-lipoic and at least about 50 ppm of 1-carnitine in the diet.

**13.** A diet for use in accordance with any preceding claim, wherein the use comprises increasing the learning ability of an aged companion pet.

**Patentansprüche**

**1.** Haustiernahrung, die die gewöhnlichen Ernähungsanforderungen für ältere Tiere erfüllt und die weiterhin eine ausreichende Menge an Antioxidans oder Mischung davon umfasst, zur Verwendung zur Inhibierung der Verschlechterung der geistigen Verfassung eines älteren Haustiers.

**2.** Haustiernahrung, die die gewöhnlichen Ernähungsanforderungen für ältere Tiere erfüllt und die weiterhin eine ausreichende Menge an Antioxidans oder Mischung davon umfasst, zur Verwendung zur Verbesserung der geistigen Verfassung eines älteren Haustiers.

**3.** Nahrung zur Verwendung gemäß Anspruch 1 oder Anspruch 2, wobei das Tier ein Hund ist.

**4.** Nahrung zur Verwendung gemäß Anspruch 3, wobei der Hund mindestens sieben Jahre alt ist.

**5.** Nahrung zur Verwendung gemäß Anspruch 1 oder Anspruch 2, wobei das Tier eine Katze ist.

**6.** Nahrung zur Verwendung gemäß Anspruch 5, wobei die Katze mindestens sieben Jahre alt ist.

**7.** Nahrung nach einem der vorgehenden Ansprüche, wobei das Antioxidans aus der Gruppe bestehend aus Vitamin C, alpha-Liponsäure, 1-Carnitin oder Mischungen davon ausgewählt ist.

**8.** Nahrung zur Verwendung gemäß einem der vorgehenden Ansprüche, wobei Vitamin E in der Nahrung in einer Menge von mindestens etwa 100 ppm der Nahrung vorhanden ist.

**9.** Nahrung zur Verwendung gemäß Anspruch 7 oder Anspruch 8, wobei mindestens etwa 50 ppm Vitamin C in der Nahrung vorhanden sind.

**10.** Nahrung zur Verwendung gemäß einem der Ansprüche 7 bis 9, wobei mindestens etwa 25 ppm Liponsäure in der Nahrung vorhanden sind.

**11.** Nahrung zur Verwendung gemäß einem der Ansprüche 7 bis 10, wobei mindestens etwa 50 ppm 1-Carnitin in der Nahrung vorhanden sind.

**12.** Nahrung zur Verwendung einem der Ansprüche 7 bis 11, die mindestens etwa 100 ppm Vitamin E, mindestens etwa 50 ppm Vitamin C, mindestens etwa 25 ppm Liponsäure und mindestens etwa 50 ppm 1-Carnitin umfasst.

**13.** Nahrung zur Verwendung gemäß einem der vorgebenden Ansprüche, wobei die Verwendung die Verbesserung der Lernfähigkeit des älteren Haustiere umfasst.


## Revendications

**1.** Régime alimentaire pour animal domestique de compagnie répondant aux exigences nutritionnelles ordinaires d'un animal domestique âgé, le régime alimentaire comprenant en outre une quantité suffisante d'un antioxydant ou un mélange de celui-ci, destiné à être utilisé pour inhiber la détérioration de la capacité mentale d'un animal domestique de compagnie âgé.

**2.** Régime alimentaire pour animal domestique de compagnie répondant aux exigences nutritionnelles ordinaires d'un animal domestique âge, le régime alimentaire comprenant en outre une quantité suffisante d'un antioxydant ou un mélange de celui-ci, destiné à être utilisé pour augmenter la capacité mentale d'un animal domestique de compagnie âgé.

**3.** Régime alimentaire destiné à être utilisé selon la revendication 1 ou 2, ou l'animal domestique est un canidé.

**4.** Régime alimentaire destiné à être utilisé selon la revendication 3, où le canidé est âgé d'au moins sept ans.

**5.** Régime alimentaire destiné à être utilisé selon la revendication 1 ou 2, où l'animal domestique est un félin.

**6.** Régime alimentaire destiné à être utilisé selon la revendication 5, où le félin est âgé d'au moins sept ans.

**7.** Régime alimentaire selon l'une quelconque des revendications précédentes, dans lequel l'antioxydant est sélectionné dans le groupe comprenant la vitamine C, l'acide alpha-lipoïque, la 1-carnitine ou des mélanges de ceux-ci.

**8.** Régime alimentaire destiné à être utilisé selon l'une quelconque des revendications précédentes, où de la vitamine E est présente dans le régime alimentaire en au moins environ 100 ppm du régime alimentaire.

**9.** Régime alimentaire destiné à être utilisé selon la revendication 7 ou 8, dans lequel au moins environ 50 ppm de vitamine C sont présents dans le régime alimentaire.

**10.** Régime alimentaire destiné à être utilisé selon l'une quelconque des revendications 7 à 9, dans lequel au moins environ 25 ppm d'acide alpha-lipoïque sont présents dans le régime alimentaire.

**11.** Régime alimentaire destiné à être utilisé selon l'une quelconque des revendications 7 à 10, dans lequel au moins environ 50 ppm de 1-carnitine sont présents dans le régime alimentaire.

**12.** Régime alimentaire destiné à être utilisé selon l'une quelconque des revendications 7 à 11, le régime alimentaire comprenant au moins environ 100 ppm de vitamine E, au moins environ 50 ppm de vitamine C, au moins environ 25 ppm d'acide alpha-lipoïque et au moins environ 50 ppm de 1-carnitine dans le régime alimentaire.

**13.** Régime alimentaire destiné à être utilisé selon l'une quelconque des revendications précédentes, son utilisation comprenant l'augmentation de la capacité d'apprentissage d'un animal domestique de compagnie âgé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0044375 A

- US 5621117 A

### Non-patent literature cited in the description

- **CUMMINGS BJ ; HEAD E ; RUEHL W ; MILGRAM NW ; COTMAN CW.** The canine as an animal model of human aging and dementia. *Neurobiology of aging,* 1996, vol. 17, 259-268

- **MILGRAM, N.W. ; ADAMS, B. ; CALLAHAN, H. ; HEAD, E. ; MACKAY, B. ; THIRLWELL, C. ; COTMAN (1999), C.W.** Landmark Discrimination Learning in the Dog. *Learning & Memory,* 1999, vol. 6, 54-61